# EUROPEAN PATENT APPLICATION

(11) **EP 4 413 982 A1**
(43) Date of publication of application: **14.08.2024**
(21) Application number: 21959865.3
(22) Date of filing: 05.10.2021
(51) Int. Cl.: A61K 31/355, A23L 33/15, A61K 31/122, A61P 27/00, A61P 27/02

(54) **COMPOSITION FOR VISUAL-ACUITY IMPROVEMENT**

(71) Applicant: Algae Health Sciences Co., Ltd., Kunming, Yunnan Province 652200 (CN)
(72) Inventor: SEKIKAWA, Takahiro, Tokyo 104-0061 (JP); KIZAWA, Yuki, Tokyo 104-0061 (JP)
(74) Representative: HGF
(86) International application number: PCT/JP2021/036853
(87) International publication number: WO 2023/058123

(57) **Abstract**

A composition for improving vision selected from more than one of uncorrected vision, corrected vision, and practical vision, containing a component (A) or the component (A) and a component (B). The component (A) is selected from at least one of Astaxanthin (ASX) or an ester of the Astaxanthin; and the component (B) is Vitamin E (VE).

## Description

### Technical Field

The present disclosure relates to a composition that can improve the vision selected from more than one of corrected vision, uncorrected vision, and practical vision.

### Background

Astaxanthin (ASX) has always been used as pigments for food additives or as colorant for farmed fish. Since the ASX was found to have 1000 times more antioxidant activity than vitamin E (VE), it has been used in pharmaceuticals, health foods such as supplements, as well as in basic cosmetics.

For example, a non-patent document 1 disclosed ASX-containing food, which has an eye adjustment function and is effective for eye fatigue. However, the non-patent document 1 does not describe astaxanthin as having vision-improving effect.

### Prior art document

### Non-patent document

Non-patent document 1: Ophthalmology Clinical Summary( 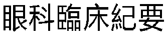 ) 3(5):2010 Pages 461-468.

### Summary

The present disclosure provides a composition for improving vision, especially improving corrected vision, uncorrected vision, and/or practical vision.

The inventor and others have carried out intensive research to solve the above problems. It was found that corrected vision and uncorrected vision can be improved by taking in an Astaxanthins (ASXs)-containing composition; and it was further found that corrected vision, uncorrected vision, and practical vision can be improved by taking in an ASXs-containing and Vitamin E (VE) composition, thereby completing the present disclosure.

That is, the present disclosure includes the following solutions.
[1] A composition for improving vision contains a component (A) or the component (A) and a component (B);
   the component (A) is selected from at least one of astaxanthin and esters of the astaxanthin, and
   the component (B) is Vitamin E (VE).
[2] In the composition as described in [1], the Vitamin E is tocotrienols.
[3] In the composition as described in [1] or [2], the composition contains the component (A), and vision is corrected vision and/or uncorrected vision.
[4] In the composition as described in [1] or [2], the composition contains the components (A) and (B), and vision is corrected vision, uncorrected vision, and/or practical vision.
[5] In the composition as described in any one of [1] to [4], the content of the component (A) is 0.1 wt% to 35wt%.
[6] In the composition as described in any one of [1] to [5], the ester of the astaxanthin includes monoester and diester, and a mass ratio (monoester/diester) of the content of the monoester to the diester is 2 to 25.
[7] The composition as described in any one of [1] to [6] is prepared in a manner that the component (A) is taken at a dose of 3 mg to 15 mg per day.
[8] The composition as described in any one of [2] to [7] is prepared in a manner that the component (B) is taken in at a dose of 30 mg to 70 mg per day.
[9] In the composition as described in any one of [1] to [8], the intake is oral.
[10] The composition as described in any one of [1] to [8] is food.
[11] The composition as described in any one of [1] to [8] is a food additive.
[12]A method for improve vision of a subject includes making a subject to take in a component (A) or the component (A) and a component (B);
   the component (A) is selected from at least one of astaxanthin and esters of the astaxanthin, and
   the component (B) is Vitamin E.
[13]An application of a component (A) or the component (A) and a component (B) in preparation of composition for improving vision;
   the component (A) is selected from at least one of astaxanthin and esters of the astaxanthin, and
   the component (B) is Vitamin E.

### Effects of the Disclosure

According to the present disclosure, the composition having the effect of improving corrected vision, uncorrected vision, and/or practical vision can be provided.

### Brief Description of the Drawings

Fig. 1 shows the dominant eye vision after Analysis of Covariance (ANCOVA) in corrected vision (logarithmic vision) after a Visual Display Terminal (VDT) workload before intake of astaxanthin (ASX) and after 6 weeks of intake.
Fig. 2 is a diagram showing variation in vision of a dominant eye after a Student's t-test in corrected vision (logarithmic vision) after a VDT workload before intake of astaxanthin (ASX) and after 6 weeks of intake.
Fig. 3 is a diagram showing vision of a dominant eye after ANCOVA in corrected vision (logarithmic vision) after a VDT workload before intake of astaxanthins and Vitamin E (ASX+VE) and after 6 weeks of intake.
Fig. 4 is a diagram showing variation in vision of a dominant eye after a Student's t-test in corrected vision (logarithmic vision) after a VDT workload before intake of astaxanthins and Vitamin E (ASX+VE) and after 6 weeks of intake.
Fig. 5 is a diagram showing vision of a left eye after ANCOVA in uncorrected vision (logarithmic vision) before a VDT workload before intake of astaxanthins (ASX) and after 6 weeks of intake.
Fig. 6 is a diagram showing vision of a left eye after ANCOVA in uncorrected vision (logarithmic vision) after a VDT workload before intake of astaxanthins and Vitamin E(ASX+VE) and after 6 weeks of intake.
Fig. 7 is a diagram showing vision of a left eye after ANCOVA in practical vision after a VDT workload before intake of astaxanthins and Vitamin E (ASX+VE) and after 6 weeks of intake.
Fig. 8 is a diagram showing average vision in both eyes after ANCOVA in practical vision after a VDT workload before intake of astaxanthins and Vitamin E (ASX+VE) and after 6 weeks of intake.
Fig. 9 is a diagram showing variation in vision of a left eye after a Student's t-test in practical vision after a VDT workload before intake of astaxanthins and Vitamin E (ASX+VE) and after 6 weeks of intake.
Fig. 10 is a diagram showing variation in average vision in both eyes after a Student's t-test in practical vision after a VDT workload before intake of astaxanthins and Vitamin E (ASX+VE) and after 6 weeks of intake.
Fig. 11 is a diagram showing variation in vision of a dominant eye after a Student's t-test in practical vision after a VDT workload before intake of astaxanthins and Vitamin E (ASX+VE) and after 6 weeks of intake.
Fig. 12 is a diagram showing variation in difference in vision of a left eye after a Student's t-test in difference (post load-pre load) in practical vision before and after a VDT workload before intake of astaxanthins and Vitamin E (ASX+VE) and after 6 weeks of intake.

### Detailed Description of the Embodiments

The present disclosure relates to an astaxanthins-containing composition for improving vision selected from more than one corrected vision and uncorrected vision, or an astaxanthins and Vitamin E-containing composition for improving vision selected from more than one uncorrected vision, corrected vision, and practical vision.

The Astaxanthin (astaxanthin, astaxanthine, 3, 3'-dihydroxy-β, β-carotene-4, 4'-dione) is a kind of carotenoid same as β-carotene of carrots and lycopene of tomatoes, and is an orange-red pigment substance commonly used in food that is classified as flavonoid. The astaxanthin is widely distributed in nature, and can be found in our sides, such as shells of crustaceans, the astaxanthin also exists on the surface of red sea bream, which feeds on crustaceans, and in the red part of the muscles of salmon.

The Astaxanthin has stereoisomers. Specifically, it is known that there are three stereoisomers of (3R, 3'R)-astaxanthin, (3R, 3'S)-astaxanthin, and (3S, 3'S)-astaxanthin. The ASX derived from natural substances is considered to be more abundant in the (3S, 3'S)-astaxanthin. In the descriptions of the specification, unless otherwise specified, when simply shown as "astaxanthin", refers to the free form of the astaxanthin including the three stereoisomers.

In the description of the specification, the term "astaxanthins" refers to at least one selected from the group consisting of free forms of the astaxanthin and ester froms of the astaxanthin. Esters of the astaxanthin include monoesters and/or diesters. Furthermore, the monoester and/or diester of the astaxanthin comprise respective monoester and/or diester of each of the three stereoisomers of the astaxanthin.

In the present disclosure, the astaxanthins is selected from at least one of the free form or esters of the astaxanthin. It is generally believed that, one or both of two hydroxyl groups of the ester of the astaxanthin are protected by ester bonds, so the astaxanthin ester form has higher physical stability than the free form, and is not easy to decompose by oxidation in food, beverages, and pharmaceuticals. However, when being taken into an organism, the astaxanthin esters are rapidly hydrolyzed into free form by enzymes in the organism thereby exhibits its effects.

Monoesters of the astaxanthin include monoesters esterified with fatty acid. Fatty acids forming the esters of the astaxanthin are not limited in the number of carbon atoms, and examples include fatty acids having 4 to 30 carbon atoms. In addition, the fatty acid forming the esters of the astaxanthin may be saturated or unsaturated. As a specific example of the fatty acid forming the esters of the astaxanthin, the following can be enumerated: acetic acid, lauric acid, myristic acid, pentadecanoic acid, palmitic acid, palmitoleic acid, margaric acid, elaidic acid, ricinoleic acid, petroselinic acid, vaccenic acid, eleostearic acid, punicic acid ( 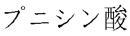 ) , licanic acid( 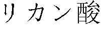 ), octadecatetraenoic acid, gadoleic acid( 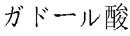 ), 5-eicosenoic acid(5 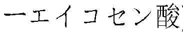 ), 5-docosaenoic acid(5 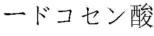 ), cetanol acid( 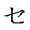 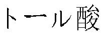 ), erucic acid, 5,13-docosadienoic acid, cis-15-tetracosenoic acid( 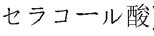 ), decenoic acid, steering acid( 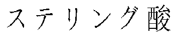 ), dodecenoic acid, oleic acid, stearic acid, eicosapentaenoic acid, docosahexaenoic acid, linoleic acid, linolenic acid, arachidonic acid, etc.

Furthermore, as monoesters of the astaxanthin, the following may be enumerated: amino acids such as glycine and aminolevulinic acid; mono or poly carboxylic acid such as acetic acid and citric acid; inorganic acids such as phosphoric acid and sulfuric acid; sugars such as glucosides; sugar fatty acids such as glycerose fatty acid( 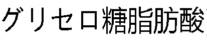 ) and glycosphingosaccharide fatty acid( 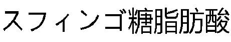 ); fatty acids such as glycerol fatty acid; and monoesters esterified by glycerophosphoric acid, etc. As the monoester of the astaxanthin, salts of the monoesters are also included.

As the diester of the astaxanthin, diesters esterified by the same or different acids selected from the fatty acids, the amino acids, the mono or poly carboxylic acid, the inorganic acids, the sugar, the sugar fatty acids, the fatty acids, and the glycerophosphoric acid can be enumerated. It is to be noted that, if possible, salts of the diesters are included.

In the present disclosure, the astaxanthins indicate those derived from natural substances or those obtained by synthesis. As astaxanthins from the natural substances, for example, astaxanthins obtained from shells, eggs, and organs of crustaceans such as shrimp, krill, and crabs; skins and eggs of various fish and shellfish; algae such as Haematococcus pluvialis; yeasts such as Phaffia rhodozyma; bacterium such as marine bacterial paracoccus; and seed plants such as amur adonis( 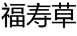 ) and buttercup( 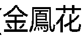 ) can be enumerated. Natural extracts and chemically-synthesized products are commercially available and easy to obtain.

Astaxanthins can be obtained by culturing astaxanthin producing microorganisms such as the Phaffia rhodozyma, the Haematococcus pluvialis, and the marine bacterial paracoccus in appropriate media in acordance with publicly-known methods. The Haematococcus pluvialis is the most suitable because of its ease of cultivation and extraction, its high concentration of astaxanthins, and its high productbility..

As long as the chlorophyta algae having the capability of producing astaxanthins, there is no particular limitation. For example, single celled algae of haematococcus genus are used preferably. As the preferred green algae, the following may be enumerated: Haematococcus pluvialis (H. pluvialis), Haematococcus lacustris (H. lacustris), Haematococcus capensis (H. capensis), Haematococcus droebakensi (H. droebakensi), Haematococcus zimbabwiensis (H. zimbabwiensis), etc.

Astaxanthins obtained from the Haematococcus pluvialis mostly includes monoester bonded with a fatty acid on a single hydroxyl group of the astaxanthin, followed by diester bonded with fatty acids at both ends, and the proportion of the presence of unmodified free form is very low. Astaxanthins obtained from the Phaffia rhodozyma mostly exists as the unmodified free form. In addition, the astaxanthins obtained from the marine bacterial paracoccus also mostly exists as the unmodified free form.

In the present disclosure, astaxanthins-containing extracts are mostly prepared as follows. Microorganisms producing astaxanthins are cultured by using a nutrient medium, and then an astaxanthins-containing oily substance is extracted. A method for extracting the astaxanthins-containing oily substance is not particularly limited, and is commonly used by those skilled in the art. For example, the cultured microorganisms producing astaxanthins are extracted through operations such as solvent suspension, drying, mechanical crushing, pressing, and supercritical extraction. These extraction operations may be performed independently, and may also be performed in combination.

As solvents for extraction, organic solvents such as chloroform, hexane, acetone, methanol and ethanol are used. The drying method can be performed according to conventional methods such as shelf drying, fluidized-bed drying, flash drying, and spray drying. The mechanical crushing method may be any one of a wet method and a dry method, and can be performed through a bead mill, a roller mill, a hammer mill, a jet mill, a pin mill, etc. The pressing method can be performed according to conventional methods. Supercritical extraction follows conventional methods to shape the crushed microorganisms producing the astaxanthins into particles, so as to extract carbon dioxide in a supercritical state or carbon dioxide in a state near a supercritical point through a layer filled with the particles, and then pressure reduction is performed to remove the carbon dioxide, so as to obtain an extract. In order to improve the extraction efficiency of astaxanthins from the particles, advanced unsaturated fatty acids, glycerol, alcohol, water, etc. may also be added.

When the solvents are used during extraction, the solvents are removed after extraction by a method commonly used by those skilled in the art. A molecular distillation apparatus can be used to further remove the solvents. Astaxanthins-containing extracts can be purified by separation column or lipases degradation as required.

The content of astaxanthins in the composition is generally 0.1 wt% or more, preferably 0.5 wt% or more, and preferably 1 wt% or more, moreover, is generally 35 wt% or less, preferably 30 wt% or less, and more preferably 25 wt% or less. The upper and lower limits above may be any combination. In addition, the content of astaxanthins is generally 0.1 wt% to 35 wt%, preferably 0.5 wt% to 30 wt%, and more preferably 1 wt% to 25 wt%.

The proportion of monoesters in astaxanthins contained in the composition is generally 40 wt% or more, preferably 50 wt% or more, more preferably 60 wt% or more, further preferably 70 wt% or more, and specially preferably 80 wt% or more. The upper limit of the content of monoesters in astaxanthins is not particularly limited, and is generally 100 wt% or less. With regard to the proportion of monoesters in astaxanthins, the upper and lower limits above may be any combination. The proportion of monoesters in astaxanthins contained in the composition is 40 wt% to 100 wt%, preferably 50 wt% to 100 wt%, more preferably 60 wt% to 100 wt%, further preferably 70 wt% to 100 wt%, and specially preferably 80 wt% to 100 wt%.

The proportion of diesters in astaxanthins contained in the composition is generally 40 wt% or less, preferably 35 wt% or less, more preferably 30 wt% or less, further preferably 25 wt% or less, and specially preferably 20 wt% or less. The lower limit of the proportion of diesters in astaxanthins contained in the composition is not particularly limited, and is generally 0 wt% or more. In addition, with regard to the proportion of diesters in astaxanthins, the upper and lower limits above may be any combination. The proportion of diesters in astaxanthins contained in the composition is generally 0 wt% to 40 wt%, preferably 0 wt% to 35 wt%, more preferably 0 wt% to 30 wt%, further preferably 0 wt% to 25 wt%, and specially preferably 0 wt% to 20 wt%.

The mass ratio (monoester/diester) of monoesters in astaxanthins contained in the composition to diesters in astaxanthins is generally 2 or more, preferably 3 or more, more preferably 4 or more, further preferably 6 or more, and specially preferably 8 or more, moreover, is generally 25 or less, preferably 20 or less, more preferably 15 or less, further preferably 13 or less, and specially preferably 10 or less. The upper and lower limits above may be any combination. The mass ratio (monoester/diester) of monoesters in astaxanthins contained in the composition to diesters in astaxanthins is generally 2 to 25, preferably 3 to 20, more preferably 4 to 15, further preferably 6 to 13, and specially preferably 8 to 10. In terms of taking both the stability of the composition and the effect in the organism into consideration, the mass ratio of monoesters in astaxanthins contained in the composition to diesters in astaxanthins is preferred within the above ranges.

Daily intake of astaxanthins is generally 3 mg or more, preferably 5 mg or more, more preferably 8 mg or more, and specially preferably 9 mg or more, moreover, is generally 15 mg or less, preferably 13 mg or less, more preferably 11 mg or less, and specially preferably 10 mg or less. The upper and lower limits above may be any combination. The daily intake of astaxanthins may generally be 3 mg to 15 mg, preferably 3 mg to 10 mg, more preferably 8 mg to 10 mg, and particular preferably 9 mg to 10 mg. In terms of fully achieving the effect of the present disclosure, the daily intake of astaxanthins is preferred within the above ranges.

The composition of the present disclosure may be astaxanthins-containing composition, and may also be an astaxanthins and vitamin E-containing composition.

In this case, astaxanthins and vitamin E may cooperate with each other. It is to be noted that a cooperation sequence of astaxanthins and vitamin E is not limited. The preferred content of astaxanthins in composition containing astaxanthins and vitamin E is described above.

The main physiological effect of the vitamin E is its antioxidant, which protects cell membranes from oxidative damage in the organism. In the present disclosure, the vitamin E is taken in with astaxanthins, so as to achieve the action of an activator for improving the effect of astaxanthins. The vitamin E is a fat-soluble vitamin, there are eight chemically different compounds according to the position and presence/absense of methyl group attached to the circulic portion in the structure. The vitamin E includes four tocopherols and four tocotrienols beginning with α-, β-, γ-, and δ-, respectively. The tocotrienols are preferably used. The tocotrienols may have higher antioxidant activity than the tocopherols.

In the present disclosure, the vitamin E can be derived from natural substances or obtained by synthesis. Tocopherols can be obtained from vegetable oil such as sunflower oil, cottonseed oil, safflower oil, rice bran oil, corn oil, rapeseed oil, soybean oil, palm oil, olive oil, perilla oil, linseed oil, sesame oil, etc. Tocotrienols can be obtained from rice, barley, wheat, rye, oil palm (palm oil), and annatto seeds. Tocotrienols are preferably obtained from the oil palm (palm oil) as they contain significant amount of all types of the α-, β-, γ-, and δ-tocotrienols .

The content of the vitamin E in composition containing astaxanthins and vitamin E is generally 10 wt% or more, preferably 14 wt% or more, and more preferably 16 wt% or more, moreover, is generally 50 wt% or less, preferably 40 wt% or less, and more preferably 30 wt% or less. The upper and lower limits above may be any combination. In addition, the content of the vitamin E is generally 10 wt% to 50 wt%, preferably 14 wt% to 40 wt%, and more preferably 16 wt% to 30 wt%.

Daily intake of the vitamin E is generally 30 mg or more, preferably 35 mg or more, more preferably 40 mg or more, and specially preferably 45 mg or more, moreover, is generally 70 mg or less, preferably 65 mg or less, more preferably 60 mg or less, and specially preferably 55 mg or less. The upper and lower limits above may be any combination. The daily intake of the vitamin E is generally 30 mg to 70 mg, preferably 35 mg to 65 mg, more preferably 40 mg to 60 mg, and particular preferably 45 mg to 55 mg.

In the specification, the daily intake refers to the amount taken by one adult per day.

The composition for improving vision in the specification is prepared in a compounding amount containing astaxanthins and/or vitamin E so that the daily intake is within a predetermined range by ingestion once to several times.

It is to be noted that, when an astaxanthins-containing material is used, astaxanthins (concentration and intake) is calculated based on the amount of the component itself in the material. In addition, when astaxanthins forms an ester or a salt, the content (concentration and intake) of astaxanthins is calculated based on values after the mass of the ester or salt is converted to the mass of equimolar free form.

Another aspect of the present disclosure provides a method for improving vision of a subject, such as human, by ingesting astaxanthins or astaxanthins and vitamin E. Therefore, more than one of corrected vision and uncorrected vision can be improved by making the subject take astaxanthins, and more than one of uncorrected vision, corrected vision, and practical vision can be improved by making the subject take astaxanthins and vitamin E. The content of astaxanthins or astaxanthins and vitamin E, and the daily intake of astaxanthins or astaxanthins and vitamin E are as described above. As an intake method, an example may be oral intake. The method of the present disclosure includes respectively and separately taking vitamin E and a free form of astaxanthins as the astaxanthin, monoester and/or diester. The method of the present disclosure generally takes 4 weeks or more, preferably 8 weeks or more, and specially preferably 12 weeks or more, to fully achieve an effect by continuously taking astaxanthins or astaxanthins and vitamin E. The age of the subject of intake is not particularly limited, for example, middle-aged and older people over 40 are preferable. It is to be noted that, ingestion methods include spontaneous intake that does not rely on medical direction.

In the present disclosure, as long as astaxanthins or astaxanthins and vitamin E are contained and the effect of the present disclosure can be achieved, the form of the composition is not particularly limited, for example, the composition can be used in any form such as liquid, paste, gel, solid, and other arbitrary forms. In addition, as long as the effect of the present disclosure can be achieved, the application form of the composition is not particularly limited, and the composition can be used in any form such as beverages, syrups, creams, jellies, powders, granules, tablets, soft capsules, hard capsules, and other arbitrary forms. In particular, the form of soft capsules is preferred in the present disclosure. Furthermore, the composition for improving vision of the present disclosure may contain other optional components such as sweeteners, spices, excipients, coloring agents, and the like within the scope of not compromising the effect of the present disclosure. In the present disclosure, the composition for improving vision can be prepared into an example form through publicly-known methods. In the present disclosure, dry products such as Phaffia rhodozyma, Haematococcus pluvialis, marine bacterial paracoccus, and the like, and crushed products thereof may be directly used with the VE together. For astaxanthins, an astaxanthins-containing extract may also be obtained through the extraction method, and is separately or together used with the vitamin E in the form of powder and an aqueous solution containing astaxanthins.

If the composition is prepared into the soft capsules, the mass of an envelope relative to the overall mass of the soft capsules is generally 35 wt% to 50 wt%, preferably 40 wt% to 45 wt%.

The composition in the present disclosure can be used as a pharmaceutical composition, a food composition, a food additive, etc.

The composition in the present disclosure can be used as capsules, solutions, suspension, emulsion, syrups, elixir( 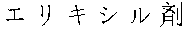 ), injection, suppository, inhalant, transnasal agents, transdermal agents, etc.

The foods are made into nutritional supplements, solid foods, fluids, beverages, and the like through conventional methods, but are not limited to these. The "food" refers to beverages and food, including all food in the form of oral. The food may also be a functional food. The functional food refers to foods with excellent physiological properties compared to ordinary foods, such as health foods, health supplements, foods for patients, nutritional supplements, or foods with health functions (foods for specific health purposes, foods with nutritional functions, and foods with functional claims), as defined by the Ministry of Health, Labor and Welfare, Japan( 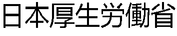 ). The composition in the present disclosure can be used as a component for health foods, health supplements, foods for patients, nutritional supplements, foods for specific health purposes, foods with nutritional functions, and foods with functional claims.

In addition, the functional claims carried by the functional food include, for example, a claim determined by a health functional food defined by the Ministry of Health, Labor and Welfare (Japan), or a claim of which content indicates improvement of vision including corrected vision, uncorrected vision, and/or practical vision. These functional claims are specifically written on, for example, a packaging container of the functional food.

In the present disclosure, the corrected vision refers to vision in a state of wearing a vision correction apparatus such as glasses or contact lenses; and the improvement of the corrected vision means that, compared with not taking the composition, the corrected vision is improved by taking the composition of the present disclosure.

In the present disclosure, the uncorrected vision refers to vision in a state without wearing the vision correction apparatus such as glasses or contact lenses; and the improvement of the uncorrected vision means that, compared with not taking the composition, the uncorrected vision is improved by taking the composition of the present disclosure.

In the present disclosure, the practical vision refers to vision required on a daily basis, taking a time factor into consideration, and refers to vision that is maintained for a certain period of time. The practical vision can be continuously measured within a period of time by using a practical vision instrument such as KOWAAS-28, all vision for which correct answers are obtained is measured, and an average value of changes in the vision is calculated for measurement. The improvement of the practical vision means that compared with not taking the composition, the practical vision is improved by taking the composition of the present disclosure.

By improving the practical vision, it is possible to maintain vision during long hours of work such as desk work and driving, and xerophthalmia(dry eye) is prevented as well.

Hereinafter, the present disclosure is described more specifically based on embodiments, but the present disclosure is not limited by these embodiments. It is to be noted that, unless otherwise specified, the units are based on mass.

The ethanol extract containing astaxanthins was prepared, and the calculated content was 5% based on free astaxanthin.

### <Preparation of astaxanthins-containing composition>

The green alga Haematococcus pluvialis was grown at 25 °C, under a light condition, a gas containing 3%CO₂, and at the same time, nutritional stress (lack of nitrogen sources) was performed on for encystment. Encapsulated cells were broken with a bead mill tissue grinder, and an astaxanthins-containing oily substance was extracted with ethanol. Vacuum concentration was performed on a crude extract to remove the ethanol, and an ethanol extract containing astaxanthins was prepared, and the calculated content was 5 wt% based on free form astaxanthin.

### <Preparation embodiment>

180 mg (9 mg of the astaxanthin) of the ethanol extract obtained through the above method and 50 mg of medium-chain fatty acid (MCT) oil were mixed, and then gelatin and glycerol were added, so as to prepare soft capsules (containing 3.26 wt% of the astaxanthin)

### (Preparation embodiment 1).

180 mg (9 mg of the astaxanthin) of the ethanol extract obtained through the above method and 50 mg of tocotrienols from rice were mixed, and then the gelatin and the glycerol were added, so as to prepare soft capsules (containing 3.26 wt% of the astaxanthin, and 18.1 wt% of the tocotrienols) (Preparation embodiment 2).

The astaxanthins contained 89.3 wt% of monoester and 9.4 wt% of diester.

In addition, 230 mg of safflower oil was prepared into soft capsules as a test food for a placebo group.

### <Subjects>

In an astaxanthins evaluation test, subjects were 22 healthy Japanese adults (an ASX group: n=12, and the placebo group: n=10), including male and female, over 40 years of age and under 64 years of age, with corrected vision being more than 1.0 in both eyes.

In an astaxanthins and vitamin E evaluation test, subjects were 21 healthy Japanese adults (an ASX and VE group: n=11, and the placebo group: n=10), including male and female, over 40 years of age and under 64 years of age, with corrected vision being more than 1.0 in both eyes.

### <Intake of test foods>

The ASX subjects took 1 soft capsule of Preparation embodiment 3 during breakfast or after breakfast for 6 weeks. The ASX and VE subjects took 1 soft capsule of Preparation embodiment 4 during breakfast or after breakfast for 6 weeks. In another aspect, subjects in the placebo group took 1 safflower oil soft capsule during breakfast or after breakfast for 6 weeks.

### <Statistical analysis>

In this test, a total of two examinations were performed at the time of trial enrollment (before test food intake) and 6 weeks from the start of intake. Measured values of measurement time points and variations from 0 week (before intake) to 6 weeks (after intake) were respectively measured between the ASX group and the placebo group, and between the ASX and VE groups, and a Student's t-test was used for evaluation. In addition, for the measured values at 6 weeks, ANCOVA was implemented by using the measured values at 0 week as covariates.

Statistical analysis was performed through a two-tailed test, and a significance level was set to 5%. Software used SPSS Ver. 23.0 for Windows (made by IBM Corporation, Japan). Without considering multiplicity due to a plurality of projects and a plurality of time points, adjustment was not performed on significance probability.

### <VDT workload checking>

In tests at 0 week and 6 weeks, VDT workload checking was implemented before and after the tests. VDT workload checking was implemented by a tester for 60 min by using Nintendo 3DS ( 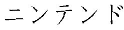 -3DS) (Tetris/ 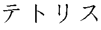 ).

### [Test embodiment 1] Corrected vision

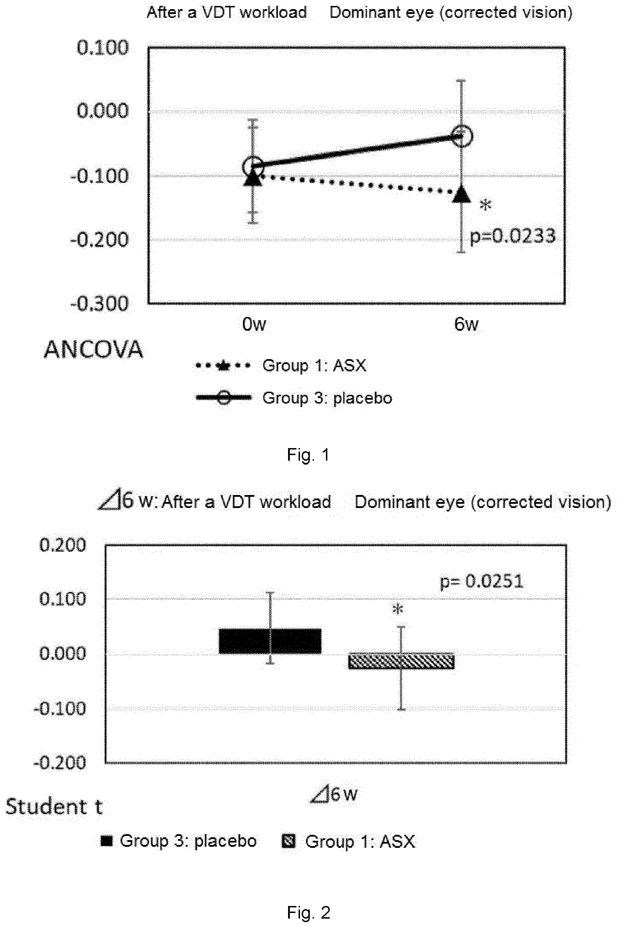

In Test embodiment 1, corrected vision was measured before test food intake and 6 weeks after test food intake in the ASX group, the ASX and VE group, and the placebo group. It is to be noted that, contact lenses and glasses for correction were not changed during test, and vision values were converted using LOGMAR values. Measured values and variations of corrected vision (logarithmic vision) were respectively shown in Figs. 1-4.

According to Figs. 1-2, it was determined that compared with the placebo group, corrected vision (logarithmic vision) of the ASX group was significantly improved after 6 weeks.

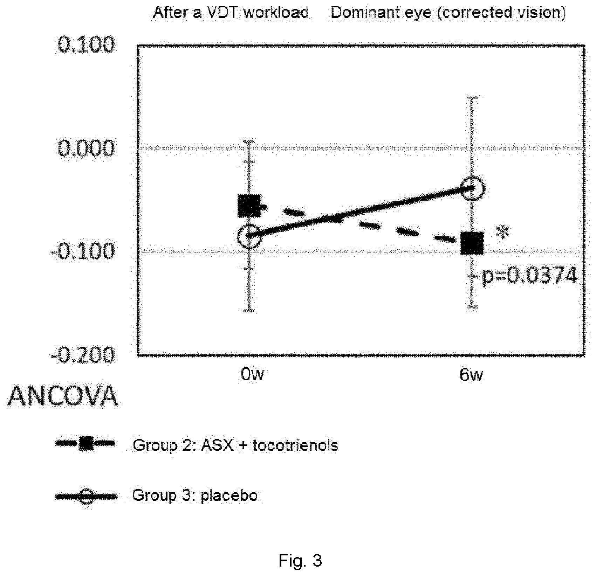

According to Figs. 3-4, it was determined that compared with the placebo group, corrected vision (logarithmic vision) of the ASX and VE group was significantly improved after 6 weeks.

### [Test embodiment 2] Uncorrected vision

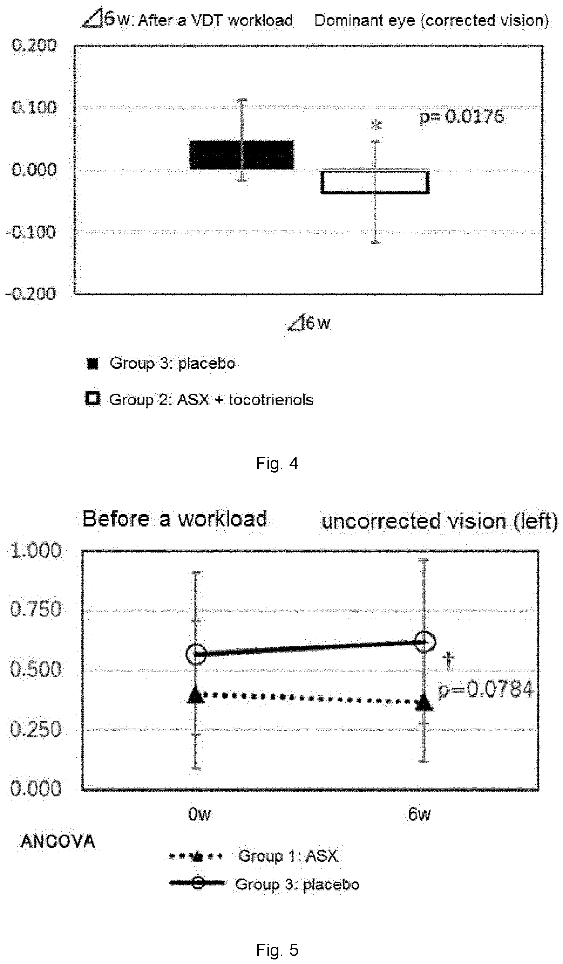

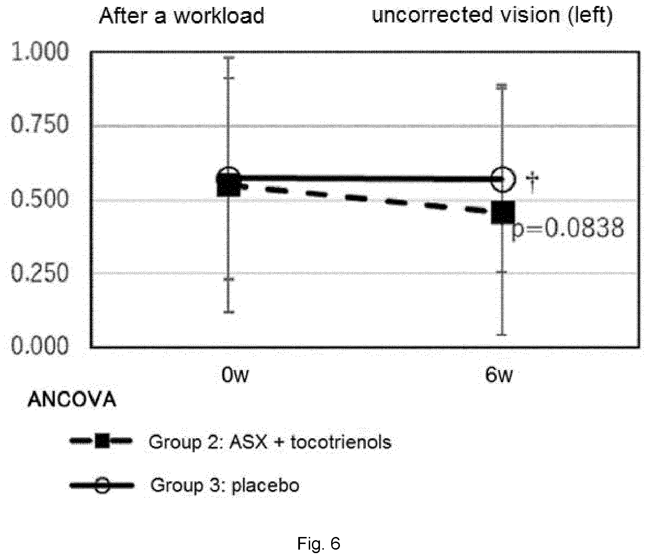

In Test embodiment 2, uncorrected vision was measured before test food intake and 6 weeks after test food intake in the ASX group, the ASX and VE group, and the placebo group. Vision values were converted using LOGMAR values. Measured values of uncorrected vision (logarithmic vision) were shown in Figs. 5-6.

According to Figs. 5-6, it might be learned that compared with the placebo group, uncorrected vision (logarithmic vision) of the ASX group and the ASX and VE group tended to improve after 6 weeks.

### [Test embodiment 3] Practical vision

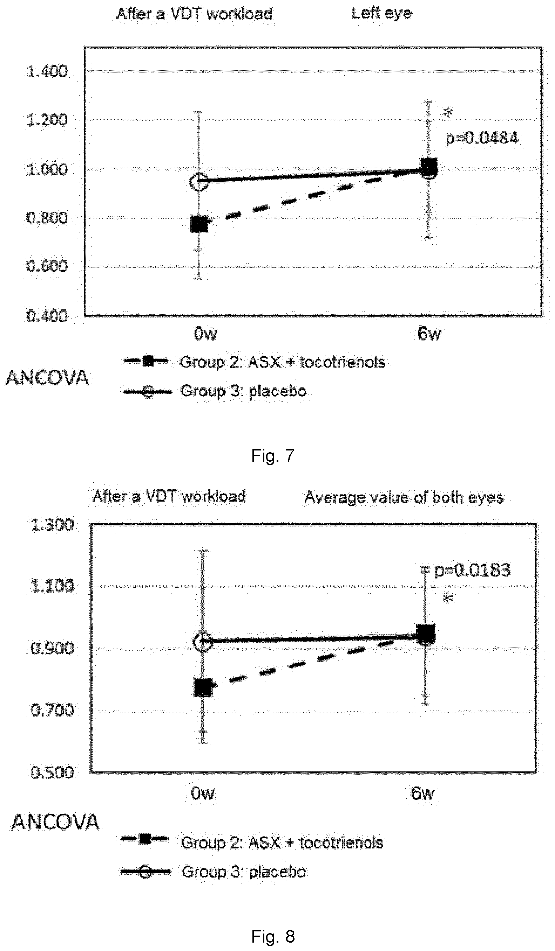

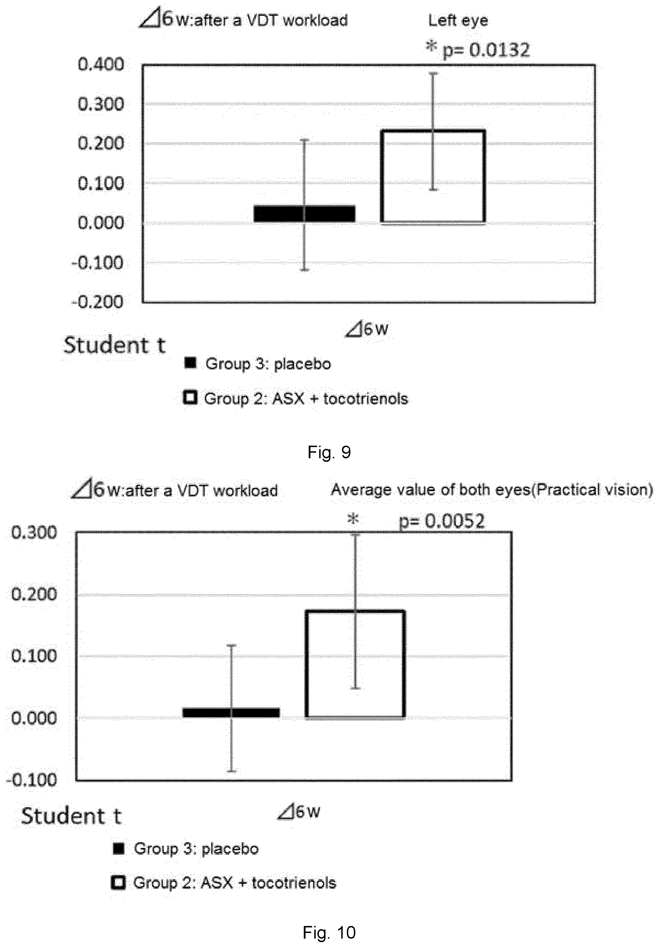

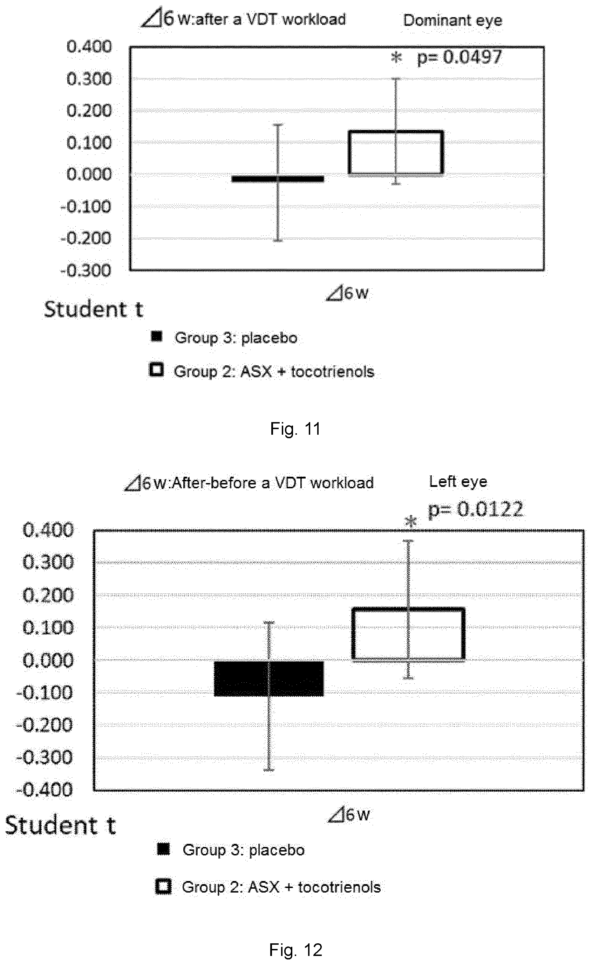

In Test embodiment 3, practical vision was measured before test food intake and 6 weeks after test food intake in the ASX and VE group, and the placebo group. Examination of practical vision used a practical peeping vision device (manufactured by Kowa, AS-28) to measure visual changes for 60 seconds. A Landolt ring( 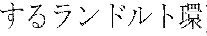 ) equal to the highest pre-measured vision was displayed and an examination was initiated, and according to correct or incorrect answers, indicators shown below were progressively changed. If the answer was correct, the indicator became small; and if the answer was incorrect, the indicator became large. The answer was considered to be incorrect if no answer was given within a certain period of time. An average value of visual changes for 60 seconds was practical vision. Measured values and variations of practical vision were shown in Figs. 7-12.

According to Figs. 7-12, it was determined that compared with the placebo group, practical vision of the ASX and VE group was significantly improved after 6 weeks.

## Claims

1. A composition for improving vision, containing a component (A) or the component (A) and a component (B), wherein
the component (A) is selected from at least one of Astaxanthin (ASX) and a group of esters of the Astaxanthin; and
the component (B) is Vitamin E (VE).

2. The composition according to claim 1, wherein the VE is tocotrienols.

3. The composition according to claim 1 or 2, wherein
the composition contains the component (A), and vision is corrected vision and/or uncorrected vision.

4. The composition according to claim 1 or 2, wherein
the composition contains the component (A) and the component (B), and vision is corrected vision, uncorrected vision, and/or practical vision.

5. The composition according to any one of claims 1 to 4, wherein
the content of the component (A) is 0.1 wt% to 35 wt%.

6. The composition according to any one of claims 1 to 5, wherein
the ester of the Astaxanthin comprises monoester and diester, and a mass ratio of the content of the monoester to the diester, i.e. monoester/diester, is 2 to 25.

7. The composition according to any one of claims 1 to 6,
prepared in a manner that the component (A) is taken in at a dose of 3 mg to 15 mg per day.

8. The composition according to any one of claims 2 to 7,
prepared in a manner that the component (B) is taken in at a dose of 30 mg to 70 mg per day.

9. The composition according to any one of claims 1 to 8, wherein
the intake is oral.

10. The composition according to any one of claims 1 to 8, wherein
the composition is food.

11. The composition according to any one of claims 1 to 8, wherein
the composition is a food additive.

12. A method for improve vision of a subject, comprising making a subject to take in a component (A) or the component (A) and a component (B), wherein
the component (A) is selected from at least one of Astaxanthin (ASX) and esters of the Astaxanthin; and
the component (B) is Vitamin E (VE).

13. An application of a component (A) or the component (A) and a component (B) in preparation of a composition for improving vision, wherein
the component (A) is selected from at least one of Astaxanthin (ASX) and esters of the Astaxanthin; and
the component (B) is Vitamin E (VE).
